# FASCICULE DE BREVET EUROPEEN

(11) **EP 2 414 099 B1**
(45) Date de publication et mention de la délivrance du brevet: **26.07.2017**
(21) Numéro de dépôt: 10713496.7
(22) Date de dépôt: 29.03.2010
(51) Int. Cl.: B01L 3/00, G01N 1/40

(54) **PROCEDE DE DETECTION ET DE QUANTIFICATION D'ANALYTES D'INTERET DANS UN LIQUIDE ET DISPOSITIF DE MISE EN OEUVRE**
VERFAHREN ZUR DETEKTION UND QUANTITATIVEN BESTIMMUNG VON ANALYTEN VON INTERESSE IN EINER FLÜSSIGKEIT UND VORRICHTUNG DAFÜR
METHOD OF DETECTING AND QUANTIFYING ANALYTES OF INTEREST IN A LIQUID AND IMPLEMENTATION DEVICE

(30) Priorité: 31.03.2009 FR 0901573
(43) Date de publication de la demande: 08.02.2012
(73) Titulaire: Centre National de la Recherche Scientifique, 75016 Paris (FR); Institut Curie, 75248 Paris Cedex 05 (FR)
(72) Inventeur: PEYRADE, Jean-Pierre, F-31520 Ramonville Saint Agne (FR); PEYRADE, David, F-38000 Grenoble (FR); VIEU, Christophe, F-31320 Auzeville Tolosane (FR); MALAQUIN, Laurent, F-91310 Linas (FR)
(74) Mandataire: Gevers & Orès
(86) Numéro de dépôt international: PCT/FR2010/000263
(87) Numéro de publication internationale: WO 2010/112699

(56) Documents cités:
- EP-A2- 1 582 855
- US-A- 5 334 837
- US-A1- 2004 043 494
- US-A1- 2004 048 388
- US-A1- 2004 203 175
- US-A1- 2004 226 885
- US-A1- 2005 079 517
- US-A1- 2006 105 453
- L. MALAQUIN ET AL: "controlled particle placement through convective and capillary assembly", LANGMUIR, vol. 23, 10 February 2007 (2007-02-10), pages 11513-11521,

## Description

L'invention se rapporte à un procédé de détection et de quantification d'analytes d'intérêt dans un liquide et à un dispositif de mise en oeuvre.

Dans la suite de la présente description, on appellera « cibles » les analytes d'intérêt recherchés.

Le procédé selon l'invention permet d'atteindre des limites de résolution allant jusqu'à cinq nano-objets par millilitre, ou inférieures à une concentration de molécules de l'atto-molaire. Le liquide peut être complexe, c'est-à-dire qu'il peut comprendre plusieurs types d'analytes, dont une partie seulement présente l'intérêt d'être détectée.

De nombreux travaux montrent que l'utilisation croissante de pesticides (fongicides, insecticides, herbicides), de raticides, d'anti-limaces, d'engrais, de détergents, d'hormones et de médicaments, disperse les molécules entrant dans leur composition dans l'environnement et donc dans les plantes et les aliments.

De même, les réactions de combustions des carburants rejettent dans l'atmosphère de nombreuses nanoparticules, de plomb et de graphite.

Les applications programmées des nanoparticules fonctionnelles posent aussi le problème de leur dispersion dans la nature.

Évaluer les risques de ces molécules et nanoparticules pour l'environnement et pour l'Homme demande de les détecter, de comprendre leur mobilité, leur réactivité, leur écotoxicité et leur persistance. Les paramètres importants à maîtriser sont surtout la détection de leur présence et le suivi de l'évolution de cette présence au cours du temps.

En effet, pouvoir détecter et analyser les analytes présents dans l'eau l'air, le sol, les plantes ou les aliments est un enjeu environnemental important.

Il s'agit également d'un enjeu médical. Pouvoir obtenir rapidement la composition des prélèvements humains du type sérums, sang ou organes, capturer des brins d'ADN, des protéines et des biomarqueurs circulants (les biomarqueurs circulants sont les précurseurs des récidives métastasiques des cancers), capturer des bactéries ou des virus, détecter des spores, à un niveau de concentration très faible, est essentiel pour établir le diagnostic médical et est la base du traitement précoce des maladies. La surveillance permanente de leur évolution est indispensable pour mesurer l'efficacité des traitements médicaux et les modifier si nécessaire, et évoluer ainsi vers une médecine individualisée.

L'accès à l'analyse des séquences de l'ADN devient nécessaire pour ouvrir le développement du « prognostique » (terme se rapportant à l'identification d'anomalies génétiques qui rendent probable le développement à long terme d'une maladie).

Ainsi, la détection, par exemple, d'un nombre réduit de copies de séquences d'acides nucléiques dans un sérum, sans recours à la PCR (Polymerase Chain Reaction) pour amplifier leur nombre, est aussi un enjeu majeur pour l'analyse médicale ou la virologie, afin de réduire les temps d'analyse.

Il est donc essentiel de disposer de techniques de mesure spécifiques et très sensibles permettant de détecter simplement, rapidement, en routine, *in situ* et à bas coût la présence de traces d'une multitude d'analytes (molécules spécifiques, nano ou micro particules, bactéries, virus, protéines, biomarqueurs circulants, ADN, spores....) dispersés dans un milieu qui peut être complexe. Ces analytes sont, par exemple, les 33 substances dangereuses énoncées dans la loi sur l'eau (Directives 76/464/CEE et 200/60/CEE) dont 50% sont à éliminer prioritairement.

Cette directive cadre sur l'eau a impulsé le développement du secteur de l'analyse de traces dans les laboratoires mais peu de techniques sont proposées au niveau industriel.

Une étude publiée par l'Agence Française de la Sécurité Sanitaire de l'Environnement et du Travail (AFSSET) (2006) intitulée « les nano-matériaux : effets sur la santé de l'homme et sur l'environnement» fait le point sur l'état de l'art en matière de techniques et de limites de détection de nano-particules dans les aérosols (gaz d'échappement, fumées) dans l'air, dans l'eau et dans les sols.

Une première catégorie est composée de techniques de détection non spécifiques (c'est-à-dire qu'elles caractérisent globalement l'ensemble des particules) et qui procèdent sans concentration des analytes préalablement à l'analyse. Il s'agit des techniques dites « Compteurs de Noyaux de Condensation » (CNC) commercialisées par la société américaine TSI et par la société allemande GRIMM. Elles consistent à détecter optiquement un nombre de particules par seconde. Les particules peuvent avoir une taille comprise entre 5nm et 1µm et une concentration comprise entre 0,1 et 10⁶ particules/ml. Elles s'appliquent surtout dans le cas de l'air et des aérosols.

La deuxième catégorie est composée de techniques de détection non spécifiques mais procédant par concentration ou accumulation des analytes préalablement à l'analyse. Ce sont des batteries de diffusion dans lesquelles le passage de l'aérosol à travers une succession de grilles classe les analytes par taille. Un couplage avec un compteur de particules permet de décompter la proportion de chaque granulométrie d'analytes dont la taille est comprise entre 2 et 200 nm. La technique ELPI (Electrical Low Pressure Impactor) sélectionne les particules chargées préalablement, par inertie, dont la taille est comprise entre 7 nm et 10 µm puis les détecte électriquement. Leur concentration doit être assez élevée (entre 1000 et 10 000 particules par ml). L'analyse de mobilité électrique (Scanning Mobility Particule Sizer ou SMPS) permet de détecter des particules de 3 à 50 nm de diamètre avec un analyseur de mobilité différentielle (DMA) et un compteur de particules.

Une autre catégorie est composée de techniques de détection spécifiques, procédant généralement par concentration préalablement à l'analyse. Des particules atmosphériques ou de sol, dont de taille est comprise entre 30 et 300 nm, sont placées en solution puis analysées, soit par chromatographie ionique, soit par spectrométrie de masse. Une autre technique utilise la fluorescence induite par laser (LIBS). Enfin, la fixation préalable de nano-traceurs reconnaissant la surface active de certaines nano-particules permet de les détecter spécifiquement.

On peut citer, dans le cas des solutions aqueuses de particules organiques, les techniques procédant par comptage des analytes par des méthodes de microscopie (microscopies électronique ou à force atomique), par séparation en taille (l'ultra filtration, la centrifugation, le fractionnement par écoulement), par chromatographie, par couplage du fractionnement avec la technique de détection dite Inductive Coupled Plasma-Mass Spectrometry (ICP-MS), et par détection laser.

On peut aussi citer, dans le cas des nanoparticules inorganiques, les techniques d'observation au microscope électronique à balayage (MEB), de la diffusion de la lumière, de la diffusion des rayons X ou des neutrons, de la « Flow Field Flow Fractionation » (FFFF), de la chromathographie hydrodynamique (HDC), de l'électrospray et de la mobilité électrique, du potentiel zeta (ou mesure de la charge de surface), de l'absorption atomique et de l'analyse des éléments légers C-H-N-S.

Cependant, ces techniques ne sont adaptées qu'à des analyses de tailles, de formes, d'état d'agrégation, de charge de surface et de composition chimique en éléments légers. Lorsqu'elles sont spécifiques, leurs limites de détection, après concentration, sont mauvaises et s'étendent de 1g/l à 10ng/l (ou 10⁸ à 10⁹ nanoparticules/ml).

Elles sont difficilement quantitatives, insuffisamment sensibles (c'est-à-dire que leur concentration limite de détection n'est pas suffisamment faible) et surtout insuffisamment spécifiques aux particules que l'on souhaite détecter généralement dans un milieu complexe. Elles sont aussi trop coûteuses, non portables sur le terrain, impraticables en routine et nécessitent des opérateurs hautement spécialisés dans des laboratoires.

Les publications relatives à la détection de molécules issues du vivant (ou biodiagnostic) donnent une limite de détection beaucoup plus poussée. Par exemple, dans « Nanostructtues in biodiagnostics » (N Chem. Rev.105 (2005) 1547-1562), un premier tableau donne les limites de détection atteintes pour l'acide nucléique. Elles fluctuent entre le nano-Molaire (nM, ou 10⁻⁹ moles par litre) et le femto Molaire (fM, ou 10⁻¹⁵ moles par litre), suivant la technique utilisée. Une seule méthode utilisant les produits d'une réaction PCR permet d'atteindre 0,1 fM. Mais cette méthode est longue en raison du temps nécessaire pour multiplier les chaines nucléotidiques. Un deuxième tableau donne les limites de détection des protéines, qui fluctuent elles aussi entre le nM et le fM. Seule la technique d'amplification par code barre permet d'atteindre 0.030 fM (30 aM) mais elle est complexe à mettre en oeuvre.

Cet examen montre qu'il existe bien une grande diversité de techniques de détection et d'analyse de solutions. Les « plus sensibles » sont développées en laboratoire, sont coûteuses, lentes, peu portables et souvent non spécifiques. Pour être crédible sur les plans de la pollution de l'environnement et du bio-diagnostique, toute technique de détection de traces doit être à bas coût, rapide, portable, sélective, suffisamment résolue pour permettre d'atteindre et si possible être au dessous du seuil de détection de 10⁵ à 10⁶ nano objets par millilitre, soit si les analytes sont des molécules, le femto-molaire. US2005/0079517, US5334837, US2004/0203175 et US20060105453 montrent des exemples de préparation d'échantillons par évaporation contrôlée. L'objectif général de la présente invention est de fournir une méthode de détection et de quantification d'analytes présents dans une solution complexe, portable, rapide et sélective, pouvant être utilisée avec de nombreux types d'analytes, économique et permettant d'atteindre, voire de dépasser, un seuil de détection de 6.10⁵ nano ou micro objets par millilitre soit, si les analytes sont des molécules, de l'ordre du femtomolaire.

Plus particulièrement, l'invention a pour objectif de permettre la détection d'analytes nano- ou micro-particulaires, d'analytes moléculaires spécifiques et de nucléotides dans une solution simple ou complexe. L'invention vise également à permettre la détection d'analytes de types spores, virus ou bactéries, dans une solution simple ou complexe. La solution complexe peut être pré-conditionnée afin de représenter les analytes présents dans l'air, l'eau, le sol, les aliments, les organismes vivants, etc.

La présente invention propose un procédé de détection d'analytes cibles d'intérêt, présents ou placés en solution, combinant une phase de concentration naturelle des analytes d'intérêt cibles dans l'échantillon et une phase de capture des analytes d'intérêt par un assemblage convectif et capillaire dirigé sur une surface munie de sondes et comprenant une phase d'analyse de la surface structurée. Ces sondes sont des motifs topographiques, chimiques, biologiques, électrostatiques ou magnétiques.

A cette fin, l'invention a pour objet un procédé tel que défini dans la revendication 1. Par convention, les étapes du procédé précédent sont faites dans l'ordre alphabétique.

Pour atteindre une grande sensibilité, c'est-à-dire la possibilité de détecter une très faible concentration d'analytes cibles, l'invention propose d'utiliser la combinaison d'une technique, efficace et reproductible, de concentration naturelle de la solution mère, avec un piégeage spécifique des analytes cibles d'intérêt en des sites organisés, appelés « sondes », disposés à la surface d'un substrat, ce qui permet une analyse automatisée de la surface du substrat.

Ainsi, l'évaporation contrôlée de l'échantillon au voisinage de la ligne triple génère des courants de convection qui concentrent les analytes dans son voisinage. En outre, lorsque la ligne triple se déplace, au fur et à mesure que le liquide s'évapore dans l'atmosphère, sur la surface structurée du substrat, les analytes cibles sont plaqués par les forces capillaires vers la surface structurée puis capturés (immobilisés) par les forces spécifiques exercées par les sondes.

Selon d'autres modes de réalisation :
- l'étape d) peut être mise en oeuvre en comptant les analytes capturés à l'étape c) par la surface micro ou nano-structurée du substrat, et en comparant le nombre d'analytes cibles capturés à une table de conversion, pour obtenir la concentration d'analytes dans la solution mère ;
- l'étape d) peut être mise en oeuvre en comptant les analytes capturés à l'étape c), ces analytes comprenant des analytes repères dont la concentration est connue et des analytes d'intérêt dont la concentration est inconnue mais proportionnelle à celle des analytes repères, et en comparant les proportions des analytes repères et des analytes d'intérêt ;
- l'étape b) peut comprendre, en outre, le dépôt d'une plaque au contact de l'échantillon liquide, pour encadrer celui-ci entre le substrat et la plaque ;
- la plaque et le substrat peuvent être déplacés l'un par rapport à l'autre selon une direction de translation sensiblement parallèle au substrat, pendant l'évaporation contrôlée de l'échantillon ;
- le substrat et l'échantillon peuvent être confinés dans une enceinte à atmosphère contrôlée ;
- lors de l'étape c), on peut réguler la pression partielle des composants du liquide dans l'atmosphère contrôlée ;
- l'étape c) peut être mise en oeuvre en apportant une quantité d'énergie suffisante pour provoquer et contrôler l'évaporation du liquide au niveau de la ligne triple ;
- le procédé peut comprendre une étape a) de pré-conditionnement de la solution mère ;
- le pré-conditionnement de la solution mère peut consister à éliminer de la solution mère des analytes inutiles, à rajouter de nouveaux analytes cibles, et/ou à rajouter des solvants ou de nouvelles molécules favorisant l'assemblage convectif et capillaire sur la surface structurée ;
- le procédé peut comprendre une étape préalable de préparation de la surface structurée consistant à déposer une goutte de liquide comprenant des molécules sondes, spécifiques des analytes cibles, sur la surface du substrat, pour que la goutte recouvre au moins partiellement la surface du substrat, puis à provoquer une évaporation contrôlée de la goutte au voisinage d'une ligne triple goutte/substrat/atmosphère, de telle sorte que cette ligne triple se déplace, à une vitesse contrôlée, sur la surface structurée du substrat, au fur et à mesure que le liquide s'évapore dans l'atmosphère, et que les molécules sondes se fixent sur la surface du substrat pour créer un réseau de sonde structurant la surface du substrat ;
- le procédé peut comprendre une étape intermédiaire entre l'étape c) et l'étape d), de fixation de fluorophores sur les analytes cibles capturés pour permettre un comptage par fluorimétrie lors de l'étape d) ;
- le procédé peut comprendre, en outre, entre l'étape c) et l'étape d), au moins une étape de différentiation spécifique des analytes piégés à l'étape c), en appliquant le substrat obtenu à l'issue de l'étape c) sur une ou plusieurs surfaces de capture fonctionnalisées ;
- lors de l'étape c), l'évaporation peut être contrôlée de telle sorte que la ligne triple se déplace à vitesse constante ;
- lors de l'étape c), l'évaporation peut être contrôlée de telle sorte que la ligne triple se déplace à vitesse variable ;
- les sondes de la surface peuvent définir, chacune, un réseau présentant un spectre optique, l'étape d) étant mise en oeuvre en analysant le spectre optique de chaque réseau après capture des analytes à l'étape c) ; et/ou
- les sondes de la surface peuvent être des cavités de tailles différentes, l'étape c) aboutissant à la capture des analytes cibles dans des micro ou nano gouttes de tailles différentes, piégées dans les cavités de tailles différentes, et l'étape d) étant mise en oeuvre en mesurant des variations d'intensité d'au moins une propriété physique ou chimique de chaque micro ou nano goutte, en déterminant le volume de la micro ou nano goutte « limite » qui ne comprend aucun analyte, la concentration de l'analyte dans la solution mère étant égale à 1 divisé par le volume de micro ou nano goutte limite.

L'invention se rapporte également à un ensemble de détection tel que défini dans la revendication 17. Selon d'autres modes de réalisation :
- le moyen d'analyse peut être apte à compter des analytes piégés par la surface structurée du substrat, et à comparer le nombre d'analytes obtenu précédemment à une table de conversion, pour obtenir la concentration d'analytes dans la solution mère ;
- l'ensemble de détection comprends, en outre, une plaque destinée à être disposée au contact de l'échantillon de solution pour enfermer celui-ci entre le substrat et la plaque ;
- le substrat et la plaque peuvent être montés mobiles et sensiblement parallèles l'un par rapport à l'autre selon une direction de translation ;
- le substrat et la plaque peuvent être montés mobiles l'un par rapport à l'autre selon une direction de translation, la plaque étant inclinée par rapport au substrat de manière à appliquer l'échantillon de solution sur le substrat structuré ;
- la plaque peut être flexible ;
- la plaque est fonctionnalisée et/ou structurée ;
- l'ensemble de détection peut comprendre, en outre, une enceinte à atmosphère contrôlée entourant le substrat et l'échantillon ;
- l'enceinte comprend un régulateur de pression partielle des composants du liquide dans l'atmosphère ;
- l'ensemble de détection peut comprendre, en outre, des canaux de pompage et/ou d'injection d'un flux de gaz ou de mélange gazeux ;
- l'ensemble de détection peut comprendre, en outre, un dispositif d'apport d'énergie, thermique et/ou électromagnétique ;
- le moyen de contrôle est couplé à au moins un dispositif d'observation de la ligne triple pour adapter le contrôle de l'évaporation et ajuster la vitesse de déplacement de la ligne triple, à au moins une valeur souhaitée, sur la surface structurée du substrat ;
- le régulateur de pression partielle peut être couplé à au moins un dispositif d'observation de la ligne triple pour adapter les pressions partielles des composants du liquide dans l'atmosphère et ajuster la vitesse de déplacement de la ligne triple, à au moins une valeurs souhaitées ;
- la surface du substrat peut comprendre une structuration prise parmi les structurations topographiques, biologiques, chimiques, électrostatiques, magnétiques ou une combinaison de ces structurations ;
- la solution mère peut être une solution colloïdale, une solution pré-conditionnée, une solution pré filtrée, une solution comportant des surfactants et des cibles d'étalonnage, une solution intégrant des cibles marquées par la couleur, par fluorescence ou par un code barre intégré, ou une solution intégrant des couplages cibles-sondes déjà réalisés en solutions ; et/ou
- la solution mère et/ou l'échantillon peut/peuvent comprendre plusieurs types de solvants.

D'autres caractéristiques de l'invention seront énoncées dans la description détaillée ci-après faite en référence aux figures annexées qui représentent, respectivement :
- la figure 1, une vue schématique de profil d'un échantillon déposé sur un substrat et subissant une évaporation naturelle ;
- la figure 2, une vue schématique de profil d'un premier mode de réalisation d'un dispositif selon l'invention, comprenant un moyen de contrôle de l'évaporation au voisinage de la ligne triple ;
- la figure 3, une vue schématique de profil d'un deuxième mode de réalisation d'un dispositif selon l'invention, comprenant une plaque supérieure de confinement de l'échantillon ;
- la figure 4, une vue schématique de profil d'une variante du mode de réalisation de la figure 3, comprenant une plaque supérieure de confinement de l'échantillon moins mouillante que le substrat ;
- la figure 5, une vue schématique de profil d'un troisième mode de réalisation d'un dispositif selon l'invention, comprenant une plaque supérieure mobile de confinement de l'échantillon ;
- la figure 6, une vue schématique de profil d'un quatrième mode de réalisation d'un dispositif selon l'invention, comprenant une enceinte à atmosphère contrôlée ;
- les figures 7a à 7f, des vues schématiques d'un mode de réalisation avantageux du procédé selon l'invention, comprenant deux étapes de différentiation spécifique des analytes piégés à la surface d'un substrat d'un dispositif selon l'invention ; et
- la figure 8, une vue en perspective d'un substrat de détection d'analytes après la mise en oeuvre du procédé selon l'invention.

Dans la description ci-après, les analytes peuvent être des micro objets (cellules, bactéries...), des nano-objets (nanoparticules, molécules spécifiques (médicaments, pesticides...)), des biomolécules (ADN, protéines...), ou encore des virus, des spores, etc.

La présente invention combine des techniques de microélectronique et de fonctionnalisation des surfaces avec des techniques d'assemblage convectif et capillaire pour piéger les analytes cibles recherchés, en des sites fonctionnels et spécifiques, ou sondes, implantés et organisés sur une surface. Ce piégeage organisé permet une étape simple de détection des zones de couplage sonde/cible et donc une étape simple d'analyse.

Il procède, d'une manière générale, en trois phases :
1) la fonctionnalisation d'une surface avec des sites sondes prédéfinis, densifiés et organisés par des techniques de microélectronique. Ces sondes exercent des forces spécifiques d'immobilisation pour permettre un prélèvement spécifique des cibles.
2) l'assemblage convectif et capillaire d'une solution colloïdale, éventuellement pré-conditionnée, contenant les analytes cibles, qui concentre naturellement la solution, sur la surface du substrat.
3) la détection de la proportion de sites sondes occupés par les cibles, qui donne, moyennant un étalonnage préalable, la concentration d'analytes cibles dans la solution testée.

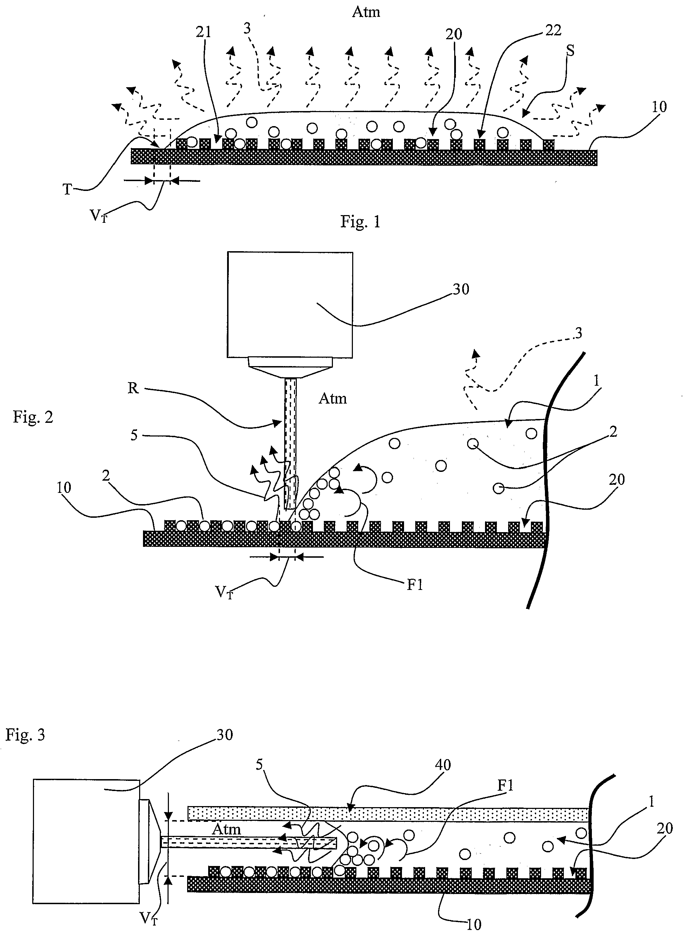

La surface fonctionnalisée comportant les sites sondes est représentée schématiquement pas des cavités 21 sur la figure 1.

Plus particulièrement, le principe du procédé selon l'invention consiste à échantillonner, c'est à dire prélever au hasard un échantillon calibré de liquide dans la solution mère à analyser. Cette solution mère présente une concentration inconnue de nano-objets à déterminer. Puis, le procédé selon l'invention consiste à concentrer et à capturer les analytes sur un substrat structuré, et à compter le nombre des analytes capturés. Ensuite, l'analyse statistique d'une ou plusieurs de ces captures permet de déduire, avec un certain intervalle de confiance, la concentration de la solution initiale. À quantité prélevée fixée, la capture contient d'autant plus d'analytes recherchés que la solution mère est concentrée. D'autre part, plus le nombre de sites de capture offerts est important plus l'analyse est représentative de la composition de la solution mère.

L'invention repose sur le contrôle de l'évaporation de l'échantillon dans une zone particulière appelée ligne triple. Cette ligne est l'interface entre le liquide de l'échantillon, l'atmosphère dans laquelle le liquide est apte à s'évaporer dans des conditions déterminées d'évaporation (de pression partielle, de température, de mouillabilité du substrat) et le substrat solide sur lequel est déposé l'échantillon. Ce contrôle permet de maîtriser le déplacement de la ligne triple sur la surface structurée du substrat (voir les figures 2 à 5).

Ainsi, le procédé selon l'invention comprend une étape b) de dépôt de l'échantillon sur un substrat de piégeage des analytes, dont au moins une partie de la surface est micro ou nano-structurée. De cette manière, l'échantillon liquide recouvre au moins partiellement la surface structurée du substrat.

Cette surface peut être structurée topographiquement, c'est-à-dire qu'elle comprend des reliefs 22 entre lesquels les analytes sont immobilisés. Mais elle peut également être structurée de manière fonctionnelle. Autrement dit, elle peut comprendre des sites de piégeage chimique, biologique, électrostatique, électrique ou magnétique. Ces sites sont obtenus, respectivement, par des sondes chimiques ou biologiques fixées sur la surface, ou par la présence de d'électrodes fournissant un champ électrostatique, électrique ou magnétique de piégeage des analytes d'intérêt. La surface peut également être structurée par des zones de mouillabilités (ou de solvabilités) différentes vis-à-vis de la solution à tester.

De préférence, la structuration de la surface est organisée selon un motif ordonné et non aléatoire, pour faciliter l'analyse automatique ultérieure du substrat.

Puis, le procédé selon l'invention comprend une étape c) d'évaporation contrôlée du liquide (ou solvant) de l'échantillon, sensiblement au niveau de la ligne triple liquide/substrat/atmosphère, de telle sorte que cette ligne triple se déplace de manière continue sur le substrat, au fur et à mesure que le liquide (ou solvant) s'évapore dans l'atmosphère.

Ce contrôle localise l'évaporation du liquide (ou solvant) sensiblement au niveau de la ligne triple. Par opposition, avec une évaporation naturelle, donc non contrôlée (symbolisée par les flèches ondulées en tirets 3 sur la figure 1), toute la surface S de liquide en contact avec l'atmosphère, et pas seulement la zone de la ligne triple liquide/substrat/atmosphère, est sujette à l'évaporation.

Bien entendu, en pratique, le contrôle de l'évaporation se fait au plus proche de la ligne triple, dans une zone de voisinage V_{T} plus large que la ligne triple. Ce qui compte, c'est que le phénomène d'évaporation du liquide (ou solvant) soit prédominant dans le voisinage V_{T} et surtout sur la ligne triple T, par rapport au reste de la surface S de l'échantillon 1 exposée à l'atmosphère.

Le procédé selon l'invention concentre naturellement, et très efficacement, les analytes présents dans l'échantillon au niveau de la ligne triple grâce à des courants de convection au sein de l'échantillon liquide. Ces courants de convection apparaissent naturellement car l'évaporation qui est prédominante sur à partir de la ligne triple demande un apport de chaleur latente, ce qui génère des échanges thermiques importants. Ces courants de convection transportent, et donc concentrent fortement, les analytes vers le voisinage V_{T} de la ligne triple T.

Puis, le procédé selon l'invention capture les analytes d'intérêt, concentrés au niveau du voisinage V_{T} de la ligne triple, sur le substrat comprenant une surface micro ou nano-structurée topographiquement, chimiquement, biologiquement, électrostatiquement, électriquement ou magnétiquement.

Le piégeage se fait lors du déplacement, continu et contrôlé, du front d'évaporation (ou ligne triple) du solvant contenant les analytes sur la surface nano-structurée. Les forces capillaires, présentes au niveau de cette ligne triple, dirigent ces analytes lors du déplacement de la ligne triple au cours de l'évaporation, vers des points précis du substrat (les sondes) définis par la structuration. Les sondes sélectionnent et immobilisent, grâce à des forces spécifiques, les analytes cibles recherchés. En d'autres termes, la ligne triple agit comme l'extrémité d'un racloir ou d'un pinceau naturel qui concentre les analytes et les étale en les plaquant dans les structures de la surface 20 du substrat.

La surface du substrat est, de préférence, traitée pour être majoritairement non mouillante (hydrophobe si le liquide est de l'eau, solvophobe si le liquide est un solvant). Cela favorise le confinement des analytes par les forces de capillarité vers les structurations de la surface du substrat.

Le nombre de structurations de piégeage des analytes, à la surface du substrat, conditionne la résolution et la sensibilité du procédé. Comme ce nombre peut être très élevé, le procédé est très sensible. Le dimensionnement des sites et/ou leur fonctionnalisation permet d'envisager des analyses sélectives par forme, charge ou fonction chimique, biologique ou magnétique. Il est possible, par exemple, de créer plus d'un million de sites sur 2 mm carrés, ce qui permet de prélever un nombre important d'analytes de l'échantillon sur le substrat, ce nombre étant directement lié à la concentration en cibles de la solution initiale.

Ainsi, le procédé selon l'invention réalise une capture organisée qui facilite l'automatisation de son dépouillement.

Ce procédé permet, au cours d'une étape d), d'analyser la surface structurée. Cette analyse peut être une détection binaire (de type 0 ou 1) rapide, et donc une mesure statistique de la concentration dans l'échantillon. La détection des captures peut être faite par optique (réflexion, contraste de phase, champ sombre, fluorescence, épifluorescence, fluorescence par cases induite (LIBS), diffraction d'un faisceau laser, plasmons de surface (SPR), utilisation de nano-traceurs), ou par champ électrostatique, électrique ou magnétique. Il est aussi envisageable de fixer, sur les cibles piégées, des particules fluorescentes puis d'analyser la surface grâce aux scanners classiques d'analyse.

Le repérage des captures d'un analyte peut être aussi obtenu en structurant individuellement les motifs sondes (par exemple en réseaux de diffraction ou en assemblages de di ou tri nanoparticules) dont le spectre optique est modifié par le piégeage.

Ainsi, les sondes de la surface micro ou nano structurée définissent, chacune, un réseau de diffraction présentant un spectre optique. L'étape d) est alors mise en oeuvre en analysant le spectre optique de chaque réseau de diffraction après capture des analytes à l'étape c).

La concentration d'analytes dans la solution mère est déduite du nombre d'analytes détectés sur le substrat, grâce à une table de conversion. Cette table associe, pour un nombre donné de structurations sur la surface du substrat, le nombre de sites occupés par les analytes à la concentration initiale dans la solution mère.

Diverses variantes sont aussi possibles pour en déduire cette concentration.

Par exemple, la concentration d'un analyte dans la solution mère peut être obtenue de manière relative, par rapport à la concentration connue d'un autre analyte, qui peut être rajouté dans la solution mère, et qui est repérable par sa couleur, par la lecture d'un code barre optique défini par des boîtes quantiques intégrées, ou par sa fluorescence. Leur proportion sur la surface fonctionnalisée du substrat donne directement la concentration de l'analyte recherché.

La concentration d'un analyte dans la solution mère peut également être obtenue en étalonnant la méthode de prélèvement dans les mêmes conditions d'évaporation à partir de différentes solutions étalons d'analytes cibles identiques.

La concentration d'un analyte dans la solution mère peut aussi être obtenue en recherchant, au préalable et dans les mêmes conditions expérimentales, la concentration seuil en analyte d'une solution étalon à partir de laquelle les premiers défauts de remplissage des sites d'accueil des cibles apparaissent.

La concentration d'un analyte dans la solution mère peut également être obtenue en déterminant, au préalable, les conditions expérimentales de piégeage des cibles, à partir de solutions étalons qui minimisent la concentration limite aboutissant à un remplissage de tous les sites. Ces conditions seront adaptées à une analyse ultrasensible.

La concentration d'un analyte dans la solution mère peut aussi être obtenue par une mesure d'intensité délivrée par chaque piège.

On peut, à cet effet, capturer les analytes avec des motifs sondes différents (en taille par exemple). On capture alors plusieurs analytes cibles par motif en piégeant, par exemple, des gouttes calibrées de liquide (éventuellement de volumes différents) sur de motifs hydrophiles ou solvophiles, séparés par des intervalles hydrophobes ou solvophobes, par un déplacement rapide de la ligne triple sur eux. L'évaporation des solvants crée également, dans ce dernier cas, des assemblages d'analytes.

Plus précisément, la surface micro ou nano structurée présente, de préférence, des cavités de tailles différentes. Le balayage rapide de cette surface par la ligne triple laisse des micro ou nano gouttes de volumes différents dans ces cavités de tailles différentes. Ainsi, plus le volume de la micro ou nano goutte est faible, moins il y a statistiquement de chance d'y trouver un analyte.

On mesure alors des variations d'intensité d'au moins une propriété physique ou chimique de chaque micro ou nano goutte. Par exemple, on mesure les variations d'intensité des propriétés optiques de chaque cavité après évaporation des micro ou nano gouttes, et on trace la courbe représentative de l'intensité en fonction du volume de la micro ou nano goutte.

Cette courbe permet de déterminer le volume de la micro ou nano goutte dite « limite » qui ne comprend aucun analyte. La concentration de l'analyte dans la solution mère est donc égale à 1 divisé par le volume de micro ou nano goutte limite.

La concentration d'un analyte dans la solution mère peut enfin être obtenue en connaissant le rendement des différentes phases de piégeage.

De préférence, le procédé selon l'invention comprend une étape de rinçage préalable à l'étape c) pour éliminer les accroches parasites.

Un exemple de calcul décrit ci-dessous permet d'évaluer les performances du procédé selon l'invention.

On réalise un substrat 10 ayant une surface de 2 mm x 2 mm présentant une structure topographique de dimensions adaptées au piégeage de nanoparticules de 100 nm de diamètre. Par exemple, la surface 20 présente des cavités 21 de 100 nm de diamètre, distantes de 2 µm. Ce substrat 10 comporte donc 10⁶ sondes sous forme de cavités de piégeage 21.

Un échantillon est déposé sur cette surface sur une épaisseur sensiblement égale au diamètre des nanoparticules qu'il contient. L'échantillon comprenant une concentration connue de 10¹¹ nanoparticules cibles par ml. Le diamètre de chaque nanoparticule est de 100 nm.

Le volume d'échantillon déposé représente donc 4.10⁻⁷ cm³ ou ml (10⁻⁵ x 4 10⁻²) et il comporte 10¹¹ x 4 10⁻⁷ = 40 000 nanoparticules.

Avec une évaporation naturelle non contrôlée du liquide (comme celle qui est illustrée à la figure 1), ces nanoparticules, supposées immobiles, ne devraient remplir, statistiquement, que les cavités face auxquelles elles sont parfaitement positionnées. Un tel prélèvement au hasard par 10⁶ cavités prélève un volume de 10⁶ x 10⁻⁵ x 10⁻⁵ x 10⁻⁵ ml sur les 4 10⁻⁷ ml de la couche, soit une nanoparticule sur 400, c'est-à-dire 100 nanoparticules (40 000/400) dans l'échantillon.

Avec une évaporation contrôlée conforme à l'invention, on observe expérimentalement qu'avec un échantillon identique, toutes les nanoparticules sont piégées par les cavités du substrat. Ainsi, les courants de convection au voisinage de la ligne triple améliorent le piégeage des nanoparticules. Il est ainsi possible de piéger avec un tel substrat, jusqu'à 10⁶ nanoparticules si la concentration de l'échantillon le permet. Le procédé selon l'invention présente donc une efficacité jusqu'à 10 000 (10⁶/100) fois supérieure à une évaporation naturelle non contrôlée.

On réalise, alors, un étalonnage avec des concentrations différentes de solution mère. Par exemple, les concentrations peuvent être de plus en plus faibles. On peut alors constituer une table de conversion entre le nombre de sites occupés par les analytes et la concentration initiale.

Le procédé selon l'invention est donc d'une grande sensibilité.

Pour atteindre la limite de détection de 6.10⁵ nanoparticules par millilitre (ou le femto molaire), il suffit donc de pouvoir détecter (10⁶/10¹¹)x(6.10⁵) assemblages, soit 6 nano-objets accrochés parmi 10⁶ motifs. Ce nombre croît à 600, si une concentration de départ de l'échantillon de 10⁹ suffisait à remplir tous les motifs. Il a ainsi été déterminé expérimentalement qu'une concentration de 5.10⁸ nano-particules par millilitre permet de combler l'ensemble de 10⁶ motifs topographiques. Une seule capture sur les 10⁶ correspondrait à une concentration d'analyte inférieure à 5 x 10⁸ / 10⁶ = 500 nano-objets par ml soit, si les nano-objets sont des molécules, à (500/6,02 10²³ ) x 10³ = 8 10⁻¹⁹ Moles/l = 0,8 atto molaire.

Avec 10⁸ sites de capture répartis sur 4 cm² la limite de détection deviendrait de 5 nano objets par ml ou 0,01 atto molaire.

Ce calcul démontre que le procédé selon l'invention permet de dépasser les limites de détection des techniques classiques, de manière simple, économique et en utilisant de très petites surfaces de capteurs, donc de faibles volumes de prélèvement. Il permet d'atteindre des limites de détection de 500 nano-objets par ml ou l'atto-molaire avec 10⁶ sites de capture répartis sur 4 mm² et 100 fois plus faibles (5 nano-objets par ml ou 0,01 atto-molaire) avec 10⁸ structurations réparties sur 4 cm².

Le procédé selon l'invention permet la détection/analyse d'analytes situés dans un environnement liquide de grande complexité (eau, sérums, etc.) mais aussi dans l'air, le sol, les aliments, après mise en suspension des analytes dans une solution mère.

Ce procédé est simple à mettre en oeuvre, rapide, économique, portable et très sensible. Il ouvre un grand champ d'applications qui s'étend depuis la nano-toxicologie, le bio diagnostic, la nano-bio médecine, la pharmacologie jusqu'à la nano-sécurité.

Des ensembles de détection d'analytes, pour la mise en oeuvre du procédé selon l'invention, sont illustrés aux figures 2 à 5.

Le mode de réalisation de la figure 2 est le plus simple. Un échantillon 1, ou goutte, comprenant des analytes d'intérêt 2, est déposé(e) sur un substrat 10 présentant une surface 20 micro ou nano-structurée (ou sondes) de piégeage des analytes cibles.

On peut favoriser l'évaporation de la goutte 1 au niveau de la ligne triple en créant un gradient en température dans le substrat.

On peut ainsi intégrer à cet ensemble un moyen de contrôle 30 de l'évaporation de l'échantillon, agencé pour provoquer une évaporation contrôlée (symbolisée par les flèches ondulées en tirets 5) de l'échantillon, sensiblement au voisinage V_{T} de la ligne triple T. Autrement dit, le moyen de contrôle favorise une évaporation dans le voisinage V_{T} de la ligne triple T qui prédomine par rapport au phénomène d'évaporation naturelle (symbolisée par les flèches ondulées en tirets 3) qui peut apparaître au niveau du reste de la surface de l'échantillon exposée à l'atmosphère Atm.

Le moyen de contrôle 30 peut, par exemple, émettre un rayonnement R, adapté et calibré pour apporter une quantité d'énergie suffisante afin de vaporiser le liquide au voisinage V_{T} de la ligne triple T. Le moyen de contrôle 30 peut, alternativement, être un flux gazeux évacuant rapidement la couche limite de liquide.

Au fur et à mesure que le liquide s'évapore dans l'atmosphère, la ligne triple T se déplace sur le substrat. Sur la figure 2, la ligne triple se déplace de la gauche vers la droite.

Pour maintenir le phénomène d'évaporation au voisinage V_{T} de la ligne triple T, le moyen de contrôle 30 peut être monté mobile en translation ou sur un pivot.

Le moyen de contrôle 30 peut également être couplé à au moins un dispositif d'observation (non illustré) de la ligne triple T pour adapter le contrôle de l'évaporation par le moyen de contrôle 30 et/ou le rayonnement émis et, ainsi réguler la vitesse de déplacement de la ligne triple, à une valeur souhaitée, sur la surface structurée du substrat. Ceci permet de contrôler la vitesse de dépôt des analytes cibles sur les sondes.

Cependant, le contrôle de l'évaporation au niveau de la ligne triple peut s'avérer délicat dans cette configuration, car l'échantillon est en milieu ouvert, c'est-à-dire qu'il présente une grande surface en contact avec l'atmosphère. En effet, les phénomènes d'évaporation naturelle peuvent, en fonction des conditions atmosphériques, jouer un rôle suffisamment important pour réduire le phénomène de concentration et donc de piégeage des analytes. De plus, l'évaporation s'effectue suivant la ligne circulaire de la goutte.

Selon un deuxième mode de réalisation illustré en figure 3, après avoir déposé l'échantillon sur le substrat, et avant de provoquer l'évaporation contrôlée de l'échantillon, le procédé selon l'invention comprend une étape de dépôt d'une plaque 40 au contact de l'échantillon liquide 1 pour encadrer celui-ci entre le substrat 10 et la plaque 40. On couvre, ainsi, la partie de la surface de liquide qui est exposée à l'atmosphère dans le dispositif précédent. La plaque est, de préférence, transparente afin de pouvoir observer le déplacement de la ligne triple et contrôler l'évaporation à son voisinage. Par exemple, elle peut être en verre lorsque le solvant de l'échantillon est de l'eau.

Cet ensemble forme une cellule micro fluidique qui permet une évaporation en milieu confiné. Autrement dit, seul le voisinage V_{T} de la ligne triple T est exposé à l'atmosphère Atm. Cet ensemble permet un meilleur contrôle de l'évaporation, ce qui conduit à une plus grande reproductibilité du remplissage des sites sondes de capture.

Le moyen de contrôle 30 provoque une évaporation qui forme un ménisque dans l'échantillon 1, entre la plaque 40 et le substrat 10, et au voisinage V_{T} de la ligne triple T. Le recul de ce ménisque, vers la droite de la figure 3, au fur et à mesure de l'évaporation, provoque un déplacement de la ligne triple T, également vers la droite de la figure 3. Dans cet ensemble, il existe également une ligne triple entre l'échantillon, l'atmosphère et la plaque 40. Cependant, cette plaque 40 n'est pas structurée, de sorte que les analytes ne se fixent pas sur la plaque 40. Elle peut être aussi structurée pour éviter que les analytes ne se fixent sur elle. Grâce aux courants de convection F1, les analytes se concentrent vers la surface structurée du substrat 10.

Le substrat 10 est traité pour n'être que partiellement mouillant et assembler les analytes vers les motifs en utilisant les forces capillaires. Les motifs sélectionnent les cibles en les fixant sous l'action de leurs forces spécifiques.

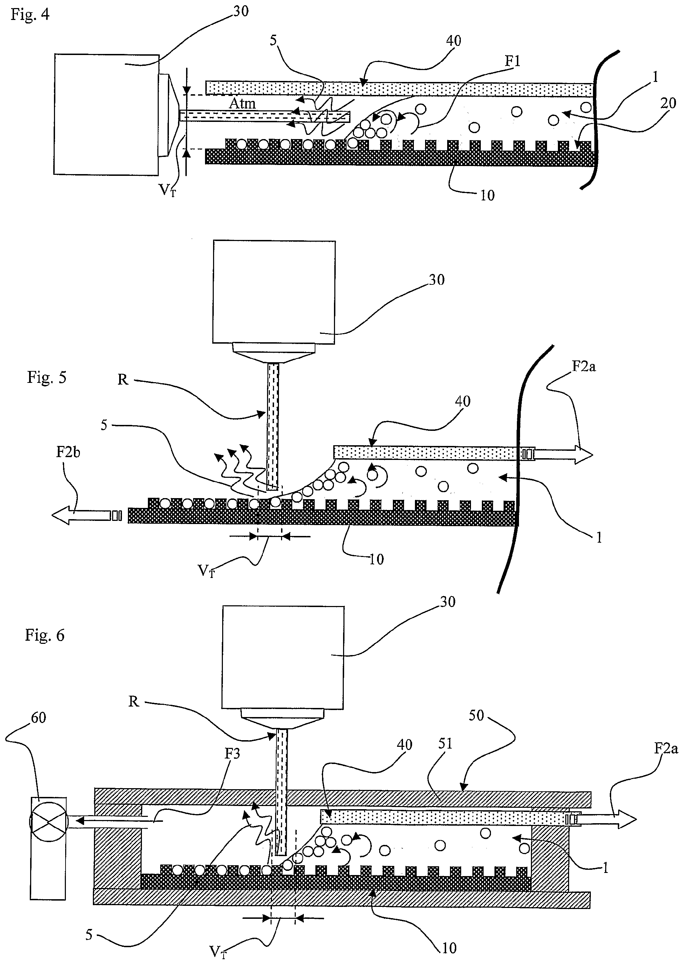

Dans le mode de réalisation illustré en figure 4, la plaque 40 a reçu un traitement superficiel qui la rend moins mouillante que le substrat. Elle tire alors la ligne triple, vers la droite de la figure 4, au fur et à mesure de l'évaporation, et dirige le déplacement de la ligne triple sur le substrat 10. L'assemblage s'effectue de manière quasi-statique.

Cet équilibre entre la mouillabilité du substrat et celle de la plaque supérieure est subtil. Le substrat ne doit pas être trop hydrophile pour éviter l'assemblage compact par convection pure entre les structurations. En cas de difficultés, une double fonctionnalisation du substrat (attraction dans les motifs et répulsion hors des motifs) sera une variante efficace. Une autre solution est de structurer la plaque.

Une variante à la cellule micro fluidique précédente, illustrée en figure 5, ajoute une translation contrôlée de la plaque supérieure 40 dans le sens de la flèche F2a. La direction de translation (flèche F2a) est sensiblement parallèle au plan du substrat 10. Cette translation présente l'avantage de contrôler l'étalement du ménisque proche de la ligne triple et donc le piégeage des analytes dans les structures de la surface 20. Alternativement ou en combinaison, la translation contrôlée peut être celle du substrat 10, dans le sens de la flèche F2b. Ce qui est important, dans ce mode de réalisation, c'est qu'un mouvement relatif soit appliqué entre le substrat 10 et la lame 40.

On peut également prévoir un dispositif de réglage de la distance entre lame supérieure 40 et le substrat 10 afin de régler la hauteur du ménisque.

La lame supérieure 40 peut être légèrement inclinée et en matériau flexible et déplacée, selon la direction de translation F2a, de manière à jouer le rôle d'un racloir (ou d'un pinceau) qui applique une solution colloïdale sur le substrat structuré.

L'inclinaison de la plaque 40 peut être réglable.

Les ensembles de détection représentés aux figures 2 à 5 peuvent être placés dans une enceinte à atmosphère contrôlée, isotherme ou à gradient de température, de manière à contrôler la vitesse de déplacement de la ligne triple.

Un exemple de mode de réalisation, illustré en figure 6, combine la cellule micro fluidique à plaque supérieure mobile 40 (et/ou à substrat 10 mobile), illustrée en figure 5, avec une enceinte 50 entourant le substrat 10, la plaque 40 et l'échantillon 1. L'enceinte 50 permet de contrôler l'atmosphère, et non plus seulement de la confiner, comme avec la cellule micro fluidique seule.

Dans un mode de réalisation plus simple (non illustré), la plaque supérieure n'est pas mobile. Elle peut alors être inutile si l'enceinte assure la même fonction en comprenant un capot 51 apte à entrer en contact avec l'échantillon 1.

L'enceinte est, de préférence, associée à un régulateur 60 de pression partielle des composants du liquide de l'échantillon (solvants et solutés) dans l'atmosphère.

Par exemple, si le solvant de l'échantillon est de l'eau, lorsque l'échantillon se vaporise au voisinage de la ligne triple, la pression partielle en eau dans l'atmosphère augmente. La cinétique du changement de phase se modifie donc, car il devient plus difficile au solvant de s'évaporer.

Le régulateur 60 peut maintenir la pression partielle au-dessous de la valeur seuil de pression de vapeur saturante de l'eau. Il commande alors une évacuation d'une partie de l'eau sous forme de vapeur (selon la flèche F3) pour que l'échantillon puisse continuer de s'évaporer. La ligne triple T se déplace alors de manière contrôlée, par cette évacuation, sur la surface 20 du substrat 10.

Le régulateur 60 peut également maintenir la pression partielle au-dessus de la valeur seuil précédente. Il bloque alors l'évacuation de l'eau sous forme de vapeur, ce qui arrête l'évaporation de l'échantillon. Le choix de la pression partielle d'eau dans l'enceinte régule donc la vitesse de déplacement de la ligne triple T sur la surface 20 du substrat 10. Dans le cas d'une solution comprenant plusieurs solvants on peut, par ce mécanisme, bloquer l'évaporation de l'un d'entre eux.

Le régulateur 60 peut également être couplé à un dispositif d'observation (non illustré) de la ligne triple T pour ajuster la vitesse de déplacement de la ligne triple à la valeur souhaitée sur la surface structurée du substrat, par le choix de la pression partielle.

Ainsi, en régulant la pression partielle de l'atmosphère, il est possible d'agir sur la vitesse de déplacement de la ligne triple sur le substrat. Ce faisant, on optimise le piégeage des analytes par la surface structurée 20 du substrat 10.

Le régulateur 60 peut également contrôler la température de l'atmosphère ou créer un gradient sur le substrat.

Un dispositif d'aspiration ou de soufflage de gaz, intégré à la plaque peut aussi contrôler la vitesse de déplacement de la ligne triple par évacuation plus rapide de la couche limite d'évaporation (évacuation des molécules de solvant vaporisées).

Il est ainsi possible de forcer, par capillarité et action de forces spécifiques, la capture de micro-objets (cellules bactéries...), de nano-objets (nanoparticules, molécules spécifiques telles que des médicaments ou des pesticides), de biomolécules (ADN, protéines...) ou encore de virus, de spores, etc.

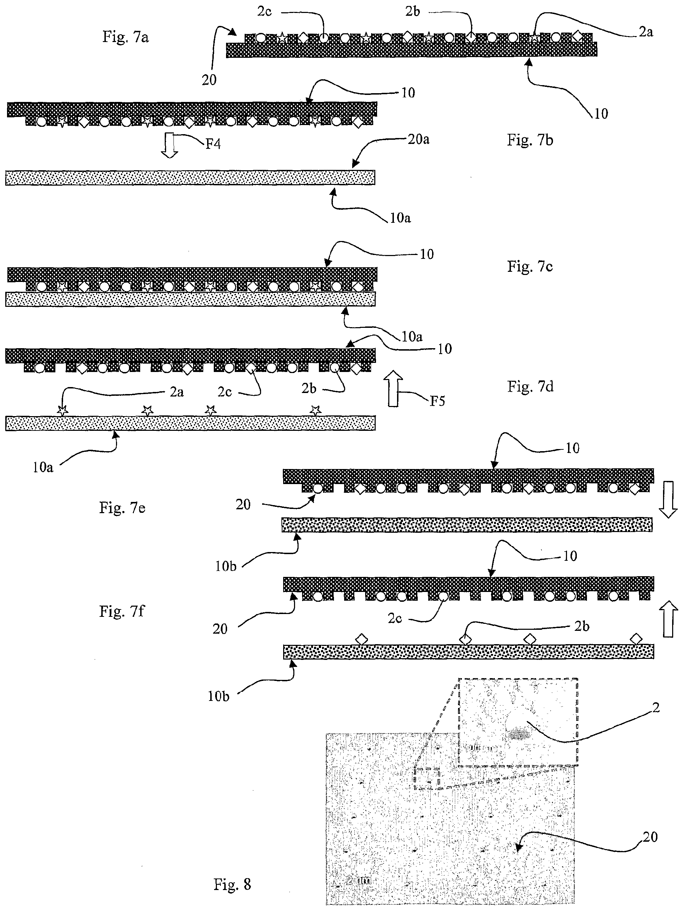

Le procédé selon l'invention peut comprendre, préalablement à l'étape c) d'analyse de la surface, une ou plusieurs étape(s) supplémentaire(s) de différentiation spécifique des analytes piégés, en appliquant le substrat obtenu à l'issue de l'étape b) sur une ou plusieurs surfaces pré-fonctionnalisées. Il est alors possible d'extraire du substrat des analytes cibles spécifiques de la surface pré-fonctionnalisée utilisée. Puis, les surfaces ainsi obtenues sont analysées conformément à l'étape c) précitée. Cette étape de différentiation spécifique des analytes piégés est illustrée aux figures 7a à 7f.

La figure 7a illustre un substrat 10 obtenu à l'issue de l'étape b). Trois types d'analytes 2a, 2b et 2c ont été capturés par la surface fonctionnalisée 20 du substrat 10.

La surface 20 du substrat 10 est appliquée, dans le sens de la flèche F4, contre un substrat 10a, muni d'une surface fonctionnalisée 20a susceptible de fixer spécifiquement les analytes 2a (figures 7b et 7c).

Puis, le substrat 10 est retiré du substrat 10a, dans le sens de la flèche F5. Le substrat 10a retire donc les analytes 2a de la surface 20 du substrat 10 (figure 7d).

L'opération est renouvelée avec un substrat 10b, muni d'une surface fonctionnalisée 20b susceptible de fixer spécifiquement les analytes 2b.

La surface 20 du substrat 10, obtenu à l'issue de l'étape de différentiation spécifique illustrée aux figures 7b à 7d, est appliquée contre le substrat 10b (figure 7e).

Puis, le substrat 10 est retiré du substrat 10b dans le sens de la flèche F5. Le substrat 10b retire donc les analytes 2b de la surface 20 du substrat 10 qui ne comprend plus que des analytes 2c.

Enfin, la surface des substrats 10, 10a et 10b ainsi obtenus est analysée conformément à l'étape c) précitée.

La fonctionnalisation des surfaces 20a-20b doit être adaptée pour que la force de transfert, c'est-à-dire d'attraction de la surface 20a- 20b soit supérieure à celle de la rétention des analytes 2a-2b dans les motifs de la surface 20 du substrat 10. Avantageusement, on utilise, pour le substrat 10, des substrats à plus faible énergie de surface, comme le PDMS, que les surfaces 20a et 20b.

Ces étape(s) supplémentaire(s) de différentiation spécifique des analytes piégés permettent une analyse automatique et spécifique de chaque type d'analytes.

La figure 8 représente la surface structurée du substrat de détection d'analytes après la mise en oeuvre du procédé selon l'invention. Sur cette figure, des nano-particules 2, de 100 nm de diamètre, sont piégées dans des cavités espacées de 2 µm sur la surface structurée 20 d'un substrat 10 puis transférées sur un substrat 10a de verre.

Le procédé selon l'invention peut être appliqué à de nombreux secteurs industriels. Plus particulièrement :
- la nano-toxicologie (c'est-à-dire la détection des nano-objets générés artificiellement par l'homme et qu'il disperse dans l'environnement) : la détection de molécules spécifiques (pesticides, médicaments, détergents...), de nano-particules ou de produits de combustion (cendres, dioxines ...) ; il permet d'étudier, éventuellement, leurs effets néfastes.
- la nano-biomédecine, c'est-à-dire l'analyse médicale, le développement de micro et de nano-techniques adaptées à la détection de plus en plus précoce d'anomalies biologiques (ADN, protéines, etc.), la détection de virus ou de bactéries, l'utilisation de nano-particules comme "vecteur" d'analyse, l'étude des milieux favorables ou défavorables à la multiplication des virus et des bactéries, etc.
- la pharmacologie pour le criblage des médicaments.
- la nano-sécurité par la détection ultrasensible de spores, de virus, de bactéries, etc.
- le traitement des surfaces et en particulier la guérison de leurs défauts superficiels.

Selon d'autres modes de réalisation :
- La fonctionnalisation de la surface du substrat peut comprendre :
   - une fonctionnalisation globale de la surface très hydrophile, appelée « assemblage convectif », pour obtenir un assemblage compact des analytes sur la surface ;
   - La fabrication de motifs topographiques de différents diamètres (pour trier les nano-objets par taille), de différentes hauteurs (pour permettre de récupérer les cibles intéressantes par tamponnage sur un autre substrat), de différents pas et géométries d'arrangement (pour mieux densifier et/ou exploiter des techniques de lecture globale) ;
   - La fabrication de motifs sondes par contrastes chimiques favorisant des interactions avec les cibles de type liaison covalente (type thiol) et/ou hydrogène et ou de Van Der Waals (radical carboné), liaison amine, liaison ionique par interaction dipôle/ dipôle, et/ou défavorisant, dans les intervalles entre motifs, ces interactions (par des contrastes chimiques de type octadecyltrimethoxysilane (OTS), amino propyltrimethoxysilane (APTES), etc., par des contrastes hydrophiles/hydrophobes-solvophiles/solvophobes) ;
   - La fabrication de motifs sondes par contrastes biologiques de type biotine, streptavidine, polyéthylène glycol, etc. ;
   - L'utilisation d'une chimie de surface passivante (comme l'OTS, le PEG - Poly Ethylène Glycol- ou la BSA - Bovine Serum Albumine-, etc.) de certaines zones pour interdire le piégeage d'anticorps, de peptides et d'ADN entre les motifs ;
   - L'injection localisée de charges positives ou négatives pour piéger des analytes polarisés ou polarisables ;
   - L'utilisation de pièges magnétiques comme des nano-particules sphériques des bâtonnets, des rubans, des tores et ou leurs empilements ;
   - La combinaison de deux ou plusieurs des procédures précédentes ;
   - L'utilisation de substrats de cibles en matériaux différents comme le verre, le silicium, l'oxyde de Silicium, le PDMS (polydimethylsiloxane), l'ITO - Indium Tin Oxyde- à forte ou à faible énergie superficielle dont les propriétés optiques comme la réflectivité préparent le dépouillement ;
   - L'utilisation de substrats ayant une surface différente de 4 mm² et comportant un nombre de sites de piégeage différent de 10⁶.
- La solution mère peut être choisie parmi :
   - Des solutions colloïdales naturelles de cibles ;
   - Des solutions à base d'eau ;
   - Des solutions à base de solvants de nature différente : organique, éther, acétone, chloroforme, octane, heptane, nonane, décane, trichloréthylène ;
   - Des prélèvements de sérums, de sang, d'organes biologiques ;
   - Des dissolutions de solides dans un solvant adéquat ;
   - Des récupérations et des pré-concentrations, par accumulation dans un solvant adapté, d'analytes présents dans l'air, dans des aérosols ou dans tout milieu complexe,
   - Des solutions dans lesquelles le piégeage cible/sonde recherché a déjà été effectué, les sondes pouvant être supportées (pré substratées) par des nanoparticules à code barre intégré (défini par des boites quantiques) défini par la couleur, la fluorescence, etc. ;
   - Des cibles marquées par des marqueurs fluorescents ;
   - Une solution complexe.
   - Des solutions pré-conditionnées par filtrage, par élimination des cibles inutiles.
- L'introduction de surfactants dans la solution mère (triton X, etc.) et de solvants (ce qui est aussi un pré conditionnement) pour faciliter l'assemblage ;
- L'introduction, dans la solution mère, d'une cible d'étalonnage en concentration et comportement connus pour permettre de déterminer des concentrations relatives.
- Le dépôt de l'échantillon de solution mère sur le substrat peut exploiter :
   - soit le caractère mouillant (hydrophile pour l'eau, solvophile pour un solvant) du substrat pour étaler la goutte déposée, soit au contraire un caractère intermédiaire mouillant/démouillant (partiellement hydrophobe pour l'eau) pour limiter son étalement en dehors de la zone structurée sur le substrat.
   - Le dépôt d'une plaque supérieure sur la goutte visant à définir une couche de liquide d'épaisseur contrôlée et à interdire l'évaporation du solvant hors de la ligne triple.
   - Cette plaque supérieure peut être fonctionnalisée non mouillante (plus hydrophobe pour l'eau que le substrat) dans le but de forcer (tirer) naturellement le déplacement de la ligne triple ;
   - Cette plaque peut être légèrement inclinée pour définir une ligne triple d'évaporation quasi rectiligne, pour former un ménisque bien défini à son extrémité et minimiser la surface de contact avec l'air.
   - Cette plaque supérieure peut être structurée
- L'étape b) d'évaporation contrôlée peut exploiter soit :
   - une évaporation naturelle plus efficace au niveau de la ligne triple que dans le reste du volume de la goutte, et qui concentre, grâce aux courants de convection, les analytes sur cette ligne ; cette évaporation naturelle peut être contrôlée en appliquant un gradient de température entre la ligne triple et le reste de la goutte ;
   - une évaporation forcée du solvant au voisinage de cette ligne triple par une réduction locale de la pression partielle du solvant (pompage), une évacuation plus rapide de la couche limite évaporée (par un flux gazeux) ou une aspiration, un chauffage, un éclairement laser, un réchauffement de la partie correspondante du substrat créant un gradient de température sur le substrat, etc ;
   - le dépôt d'une plaque au contact de la goutte de liquide qui encadre celui-ci entre le substrat et la plaque ce qui limite et confine l'évaporation dans la région proche de la ligne triple ;
- L'étape b) d'évaporation contrôlée peut être utilisée pour :
   - d'abord assembler les sondes puis renouvelée pour assembler les cibles sur ces sondes ;
   - prélever des micro ou nano gouttes de solution par déplacement rapide de la ligne triple et piéger ainsi des arrangements d'analytes ou réaliser des micro-nano prélèvements ou des micro nano laboratoires
- un dispositif du suivi du déplacement de la ligne triple peut être intégré pour permettre d'automatiser le dépôt de l'échantillon liquide sur le substrat, grâce à une électronique de contre-réaction couplée à une analyse d'image ;
- le substrat peut être d'un premier matériau et recouvert, totalement ou partiellement, de couches structurées de capture de matériaux différents.
- l'ensemble de détection d'analytes peut comprendre, en outre, des canaux de pompage et/ou d'injection d'un flux de gaz. En particulier, dans les modes de réalisations illustrés aux figures 3 à 6, la plaque 40 peut supporter ces canaux de pompage et/ou d'injection d'un flux de gaz ;
- la solution mère peut être une solution colloïdale, une solution intégrant des surfactants et des cibles d'étalonnage, une solution intégrant des cibles marquées par fluorescence ou une solution intégrant des couplages cibles-sondes déjà réalisés en solution. Dans ce dernier cas, la surface structurée du substrat 10 peut être choisie pour fixer préférentiellement ou successivement la sonde, la cible ou l'ensemble ;
- la solution mère et/ou l'échantillon peu(ven)t comprendre plusieurs solvants.

L'une des originalités de cette invention est de contrôler l'évaporation d'un solvant au voisinage de la ligne triple qui concentre naturellement les analytes cibles recherchés sur cette ligne en une organisation compacte. Ce phénomène de concentration provient de la génération de courants de convection dans le liquide qui s'évapore, dont le rôle est d'apporter l'énergie thermique (ou chaleur latente) nécessaire à la vaporisation. L'utilisation d'une évaporation contrôlée de l'échantillon, déposé sur une surface structurée, permet des analyses par taille ou par propriétés chimiques, biologiques, électrostatiques, électriques et/ou magnétique des analytes.

Une autre originalité consiste à atteindre une très faible limite de détection en effectuant le prélèvement sur un grand nombre de sites sondes d'accueil fonctionnels ou sélectifs occupant une très petite surface. L'invention est donc adaptée à l'analyse de prélèvements de quelques centièmes de microlitre.

En outre, l'organisation des sondes permet d'exploiter des techniques de détection automatisées de l'accroche sonde-cible. L'invention permet ainsi d'obtenir un motif bien défini de sites occupés et inoccupés par les analytes recherchés, et un dépouillement simple et automatisable.

L'invention propose donc un laboratoire sur puce qui accélère, et baisse les coûts des analyses, et qui permet de nombreuses analyses en parallèle.

## Revendications

1. Procédé de détection et de quantification d'analytes cibles d'intérêt (2) dans un échantillon (1) de liquide obtenu à partir d'une solution mère, ladite solution mère ayant une concentration inconnue d'analytes à déterminer, le liquide étant apte à s'évaporer dans une atmosphère (Atm) dans des conditions déterminées d'évaporation, le procédé comprenant les étapes suivantes :
b1) déposer l'échantillon (1) sur un substrat (10) présentant une surface (20) micro ou nano-structurée définissant des sondes de capture des analyte, pour que l'échantillon liquide recouvre au moins partiellement la surface structurée du substrat ;
b2) déposer une plaque (40) fonctionnalisée et/ou structurée au contact de l'échantillon liquide (1), pour encadrer celui-ci entre le substrat (10) et la plaque (40) ;
c) provoquer une évaporation contrôlée (5) de l'échantillon au voisinage (V_{T}) d'une ligne triple (T) liquide/substrat/atmosphère, constituée par l'interface entre le liquide de l'échantillon, l'atmosphère et le substrat, de telle sorte que cette ligne triple se déplace, à une vitesse contrôlée, sur la surface structurée du substrat, au fur et à mesure que le liquide s'évapore dans l'atmosphère, et que les analytes cibles soient capturés, par assemblage convectif et capillaire dirigé vers les sondes ;
d) analyser la surface structurée du substrat obtenu à l'issue de l'étape c).

2. Procédé de détection et de quantification d'analytes selon la revendication 1, dans lequel l'étape d) est mise en oeuvre en comptant les analytes capturés à l'étape c) par la surface micro ou nano-structurée du substrat, et en comparant le nombre d'analytes cibles capturés à une table de conversion, pour obtenir la concentration d'analytes dans la solution mère.

3. Procédé de détection et de quantification d'analytes relative selon la revendication 1, dans lequel l'étape d) est mise en oeuvre en comptant les analytes capturés à l'étape c), ces analytes comprenant des analytes repères dont la concentration est connue et des analytes d'intérêt dont la concentration est inconnue mais proportionnelle à celle des analytes repères, et en comparant les proportions des analytes repères et des analytes d'intérêt.

4. Procédé de détection et de quantification d'analytes selon l'une des revendications précédentes, dans lequel la plaque (40) et le subtrat (10) sont déplacés l'un par rapport à l'autre selon une direction de translation (F2a-F2b) sensiblement parallèle au substrat, pendant l'évaporation contrôlée de l'échantillon.

5. Procédé de détection et de quantification d'analytes selon l'une des revendications précédentes, dans lequel le substrat et l'échantillon sont confinés dans une enceinte (50) à atmosphère contrôlée.

6. Procédé de détection et de quantification d'analytes selon la revendication 5, dans lequel lors de l'étape c), on régule la pression partielle des composants du liquide dans l'atmosphère contrôlée.

7. Procédé de détection et de quantification d'analytes selon l'une quelconque des revendications précédentes, dans lequel l'étape c) est mise en oeuvre en apportant une quantité d'énergie suffisante pour provoquer et contrôler l'évaporation du liquide au niveau de la ligne triple.

8. Procédé de détection et de quantification d'analytes selon l'une quelconque des revendications précédentes, comprenant une étape a) de pré-conditionnement de la solution mère.

9. Procédé de détection et de quantification d'analytes selon la revendication 8, dans lequel le pré-conditionnement de la solution mère consiste à éliminer de la solution mère des analytes inutiles, à rajouter de nouveaux analytes cibles, et/ou à rajouter des solvants ou de nouvelles molécules favorisant l'assemblage convectif et capillaire sur la surface structurée.

10. Procédé de détection et de quantification d'analytes selon l'une quelconque des revendications précédentes, comprenant une étape préalable de préparation de la surface structurée consistant à déposer une goutte de liquide comprenant des molécules sondes, spécifiques des analytes cibles, sur la surface (20) du substrat (10), pour que la goutte recouvre au moins partiellement la surface du substrat, puis à provoquer une évaporation contrôlée de la goutte au voisinage (VT) d'une ligne triple (T) goutte/substrat/atmosphère, de telle sorte que cette ligne triple se déplace, à une vitesse contrôlée, sur la surface structurée du substrat, au fur et à mesure que le liquide s'évapore dans l'atmosphère, et que les molécules sondes se fixent sur la surface du substrat pour créer un réseau de sonde structurant la surface du substrat.

11. Procédé de détection et de quantification d'analytes selon l'une quelconque des revendications précédentes, comprenant une étape intermédiaire entre l'étape c) et l'étape d), de fixation de fluorophores sur les analytes cibles capturés pour permettre un comptage par fluorimétrie lors de l'étape d) ;

12. Procédé de détection et de quantification selon l'une quelconque des revendications précédentes, comprenant, en outre, entre l'étape c) et l'étape d), au moins une étape de différentiation spécifique des analytes piégés (2a, 2b) à l'étape c), en appliquant le substrat (10) obtenu à l'issue de l'étape c) sur une ou plusieurs surfaces (20a, 20b) de capture fonctionnalisées.

13. Procédé de détection et de quantification selon l'une quelconque des revendications précédentes, dans lequel, lors de l'étape c), l'évaporation est contrôlée de telle sorte que la ligne triple (T) se déplace à vitesse constante.

14. Procédé de détection et de quantification selon l'une quelconque des revendications 1 à 12, dans lequel, lors de l'étape c), l'évaporation est contrôlée de telle sorte que la ligne triple (T) se déplace à vitesse variable.

15. Procédé de détection et de quantification selon l'une quelconque des revendications précédentes, dans lequel les sondes de la surface (20) définissant, chacune, un réseau présentant un spectre optique, l'étape d) étant mise en oeuvre en analysant le spectre optique de chaque réseau après capture des analytes à l'étape c).

16. Procédé de détection et de quantification selon l'une quelconque des revendications 1 à 14, dans lequel les sondes de la surface (20) sont des cavités de tailles différentes, l'étape c) aboutissant à la capture des analytes cibles dans des micro ou nano gouttes de tailles différentes, piégées dans les cavités de tailles différentes, et l'étape d) étant mise en oeuvre en mesurant des variations d'intensité d'au moins une propriété physique ou chimique de chaque micro ou nano goutte, en déterminant le volume de la micro ou nano goutte « limite » qui ne comprend aucun analyte, la concentration de l'analyte dans la solution mère étant égale à 1 divisé par le volume de micro ou nano goutte limite.

17. Ensemble de détection et de quantification d'analytes pour la mise en oeuvre du procédé selon l'une quelconque des revendications 1 à 13, comprenant :
- un substrat (10) présentant une surface structurée destinée à recevoir un échantillon de solution mère contenant les analytes d'intérêt ;
- un moyen de contrôle (30) de l'évaporation de la solution au voisinage (V_{T}) d'une ligne triple (T) liquide/substrat/atmosphère ;
- une plaque (40) destinée à être disposée au contact de l'échantillon de solution pour enfermer celui-ci entre le substrat et la plaque ;
- un moyen d'analyse de la surface micro ou nano-structurée du substrat, **caractérisé en ce que** ladite plaque est fonctionnalisée et/ou structurée.

18. Ensemble de détection et de quantification d'analytes selon la revendication 17, **caractérisé en ce que** la plaque (40) est plus hydrophobe que le substrat (10).

19. Ensemble de détection et de quantification d'analytes selon l'une des revendications 17 ou 18, **caractérisé en ce qu'**il comporte en outre un ou plusieurs substrats (10a, 10b) comportant chacun une surface fonctionnalisée (20a, 20b) susceptible de fixer spécifiquement des analytes d'intérêt (2a, 2b).

20. Ensemble de détection et de quantification d'analytes selon l'une des revendications 17 à 19, dans lequel le moyen d'analyse est apte à compter des analytes piégés par la surface structurée du substrat.

21. Ensemble de détection et de quantification d'analytes selon l'une des revendications 17 à 20, dans lequel le moyen d'analyse est apte à comparer le nombre d'analytes obtenu précédemment à une table de conversion, pour obtenir la concentration d'analytes dans la solution mère.

22. Ensemble de détection et de quantification d'analytes selon l'une des revendications 17 à 21, dans lequel le substrat (10) et la plaque (40) sont montés mobiles et sensiblement parallèles l'un par rapport à l'autre selon une direction de translation (F2a-F2b).

23. Ensemble de détection et de quantification d'analytes selon l'une des revendications 17 à 21, dans lequel le substrat (10) et la plaque (40) sont montés mobiles l'un par rapport à l'autre selon une direction de translation (F2a-F2b), la plaque (40) étant montée inclinée par rapport au substrat (10) de manière à appliquer l'échantillon de solution sur le substrat structuré.

24. Ensemble de détection et de quantification d'analytes selon l'une des revendications 17 à 23, dans lequel la plaque (40) est flexible.

25. Ensemble de détection et de quantification d'analytes selon l'une des revendications 17 à 24, comprenant, en outre, une enceinte (50) à atmosphère contrôlée entourant le substrat et l'échantillon.

26. Ensemble de détection et de quantification d'analytes selon la revendication 25, dans lequel l'enceinte comprend un régulateur (60) de pression partielle des composants du liquide dans l'atmosphère.

27. Ensemble de détection et de quantification d'analytes selon l'une des revendications 25 ou 26, comprenant, en outre, des canaux de pompage et/ou d'injection d'un flux de gaz ou de mélange gazeux.

28. Ensemble de détection et de quantification d'analytes selon l'une quelconque des revendications 17 à 27, comprenant, en outre, un dispositif d'apport d'énergie, thermique et/ou électromagnétique.

29. Ensemble de détection et de quantification d'analytes selon l'une quelconque des revendications 17 à 28, dans lequel le moyen de contrôle (30) est couplé à au moins un dispositif d'observation de la ligne triple (T) pour adapter le contrôle de l'évaporation et ajuster la vitesse de déplacement de la ligne triple, à au moins une valeur souhaitée, sur la surface structurée du substrat.

30. Ensemble de détection et de quantification d'analytes selon l'une des revendications 26 à 29, dans lequel le régulateur de pression partielle (60) est couplé à au moins un dispositif d'observation de la ligne triple (T) pour adapter les pressions partielles des composants du liquide dans l'atmosphère et ajuster la vitesse de déplacement de la ligne triple (T), à au moins une valeurs souhaitées.

31. Ensemble de détection et de quantification d'analytes selon l'une quelconque des revendications 17 à 30, dans lequel la surface du substrat comprend une structuration prise parmi les structurations topographiques, biologiques, chimiques, électrostatiques, magnétiques ou une combinaison de ces structurations.

32. Ensemble de détection et de quantification d'analytes selon l'une quelconque des revendications 17 à 31, dans lequel la solution mère est une solution colloïdale, une solution pré-conditionnée, une solution préfiltrée, une solution comportant des surfactants et des cibles d'étalonnage, une solution intégrant des cibles marquées par la couleur, par fluorescence ou par un code barre intégré, ou une solution intégrant des couplages cibles-sondes déjà réalisés en solutions.

## Patentansprüche

1. Verfahren zur Detektion und Quantifizierung von Zielanalyten von Interesse (2) in einer Flüssigprobe (1), die aus einer Stammlösung gezogen wurde, wobei die Stammlösung eine unbekannte und zu bestimmende Konzentration von Analyten aufweist, die Flüssigkeit geeignet ist, in einer Atmosphäre (Atm) unter festgelegten Verdampfungsbedingungen zu verdampfen und das Verfahren die folgenden Schritte umfasst:
b1) Ablegen der Probe (1) auf einem Substrat (10) mit einer Oberfläche (20) mit Mikro- oder Nanostruktur mit Definition von Abfangsonden für Analyten, damit die Flüssigkeitsprobe die strukturierte Oberfläche des Substrats zumindest teilweise bedeckt;
b2) Ablegen einer funktionalisierten und/oder strukturierten Platte (40) in Berührung mit der Flüssigprobe (1), um diese zwischen dem Substrat (10) und der Platte (40) einzuschließen;
c) Auslösen einer gesteuerten Verdampfung (5) der Probe in der Nähe (V_{T}) einer dreifachen Linie (T) Flüssigkeit / Substrat / Atmosphäre, die durch die Grenzfläche zwischen der Probenflüssigkeit, der Atmosphäre und dem Substrat gebildet wird, so dass sich diese dreifache Linie mit gesteuerter Geschwindigkeit auf der strukturierten Fläche des Substrats nach und nach so bewegt, wie die Flüssigkeit in die Atmosphäre verdampft und die Zielanalyten durch konvektive und kapillare zu den Sonden gerichtete Zusammenstellung erfasst werden;
d) Auswerten der strukturierten Fläche des beim Schritt c) erzielten Substrats.

2. Verfahren zur Detektion und Quantifizierung von Analyten nach Anspruch 1, wobei der Schritt d) mit Zählen der beim Schritt c) durch die Oberfläche mit Mikro- oder Nanostruktur des Substrats erfassten Analyten eingesetzt wird und die Anzahl der erfassten Zielanalyten mit einer Umwandlungstabelle verglichen wird, um zur Analytkonzentration in der Stammlösung zu gelangen.

3. Verfahren zur Detektion und relativen Quantifizierung von Analyten nach Anspruch 1, wobei der Schritt d) mit Zählen der beim Schritt c) erfassten Analyten eingesetzt wird, diese Analyten gekennzeichnete Analyten umfassen, deren Konzentration bekannt ist, und Analyten von Interesse, deren Konzentration unbekannt ist, aber im Verhältnis zu derjenigen der gekennzeichneten Analyten ist und mit Vergleich der Verhältnisse der gekennzeichneten Analyten zu den Analyten von Interesse.

4. Verfahren zur Detektion und Quantifizierung von Analyten nach einem beliebigen der vorstehenden Ansprüche, wobei die Platte (40) und das Substrat (10) während der gesteuerten Verdampfung der Probe in einer Translationsbewegung (F2a-F2b) deutlich parallel zum Substrat verschoben werden.

5. Verfahren zur Detektion und Quantifizierung von Analyten nach einem beliebigen der vorstehenden Ansprüche, wobei das Substrat und die Probe in einer Umschließung (50) unter Schutzatmosphäre eingeschlossen sind.

6. Verfahren zur Detektion und Quantifizierung von Analyten nach Anspruch 5, wobei beim Schritt c) der Partialdruck der Komponenten der Flüssigkeit unter der Schutzatmosphäre reguliert wird.

7. Verfahren zur Detektion und Quantifizierung von Analyten nach einem beliebigen der vorstehenden Ansprüche, wobei der Schritt c) durch Bereitstellen einer Energiemenge durchgeführt wird, die ausreicht, um die Verdampfung der Flüssigkeit an der Dreifachlinie auszulösen und zu steuern.

8. Verfahren zur Detektion und Quantifizierung von Analyten nach einem beliebigen der vorstehenden Ansprüche, das einen Schritt a) der Vorkonditionierung der Stammlösung umfasst.

9. Verfahren zur Detektion und Quantifizierung von Analyten nach Anspruch 8, wobei die Vorkonditionierung der Stammlösung darin besteht, nutzlose Analyten aus der Stammlösung zu entfernen, neue Zielanalyten hinzuzufügen und/oder Lösungsmittel oder neue Moleküle hinzuzufügen, die die konvektive und kapillare Zusammenstellung auf der strukturierten Oberfläche begünstigen.

10. Verfahren zur Detektion und Quantifizierung von Analyten nach einem beliebigen der vorstehenden Ansprüche, das einen vorherigen Schritt mit Vorbereitung der strukturierten Oberfläche umfasst, der darin besteht, einen Tropfen einer für Zielanalyten spezifische Sondenmoleküle enthaltenden Flüssigkeit auf die Oberfläche (20) des Substrats (10) aufzubringen, damit der Tropfen zumindest teilweise die Oberfläche des Substrats bedeckt, und dann eine gesteuerte Verdampfung des Tropfens in der Nachbarschaft (VT) einer dreifachen Linie (T) Tropfen/Substrat/Atmosphäre auszulösen, so dass sich diese dreifache Linie mit gesteuerter Geschwindigkeit auf der strukturierten Fläche des Substrats nach und nach so bewegt, wie die Flüssigkeit in die Atmosphäre verdampft, und dass die Sondenmoleküle sich auf der Oberfläche des Substrats fixieren, um ein Sondennetz zu bilden, das die Oberfläche des Substrats strukturiert.

11. Verfahren zur Detektion und Quantifizierung von Analyten nach einem beliebigen der vorstehenden Ansprüche, das einen Zwischenschritt zwischen dem Schritt c) und dem Schritt d) mit Fixierung von Fluorophoren auf den erfassten Zielanalyten umfasst, damit beim Schritt d) ein Zählen durch Fluorimetrie möglich wird.

12. Verfahren zur Detektion und Quantifizierung nach einem beliebigen der vorstehenden Ansprüche, das ferner zwischen dem Schritt c) und dem Schritt d) zumindest einen Schritt mit spezifischer Differenzierung der beim Schritt c) gefangenen Analyten (2a, 2b) umfasst, indem das bei Ausgang des Schritts c) gewonnene Substrat (10) auf einer oder mehreren funktionalisierten Fangoberflächen (20a, 20b) aufgebracht wird.

13. Verfahren zur Detektion und Quantifizierung nach einem beliebigen der vorstehenden Ansprüche, wobei beim Schritt c) die Verdampfung so gesteuert wird, dass die dreifache Linie (T) sich bei konstanter Geschwindigkeit bewegt.

14. Verfahren zur Detektion und Quantifizierung nach einem beliebigen der vorstehenden Ansprüche 1 bis 12, wobei beim Schritt c) die Verdampfung so gesteuert wird, dass die dreifache Linie (T) sich bei variabler Geschwindigkeit bewegt.

15. Verfahren zur Detektion und Quantifizierung nach einem beliebigen der
vorstehenden Ansprüche, wobei die Sonden der Oberfläche (20) jeweils ein Netz definieren, das ein optisches Spektrum aufweist, und der Schritt d) eingesetzt wird mit Analyse des optischen Spektrums jedes Netzes nach der Erfassung der Analyten beim Schritt c).

16. Verfahren zur Detektion und Quantifizierung nach einem beliebigen der vorstehenden Ansprüche 1 bis 14, wobei die Sonden der Oberfläche (20) Hohlräume verschiedener Größen sind, der Schritt c) zum Erfassen der Zielanalyten in Mikro- oder Nanotropfen verschiedener Größen führt, die in den Hohlräumen verschiedener Größen gefangen werden, und der Schritt d) eingesetzt wird mit Messung der Intensitätsänderungen zumindest einer physikalischen oder chemischen Eigenschaft jedes Mikro- oder Nanotropfens unter Bestimmung des "grenzwertigen" Volumens des Mikro- oder Nanotropfens, der keinen Analyten enthält und die Konzentration des Analyts in der Stammlösung gleich 1 dividiert durch das Volumen des grenzwertigen Mikro- oder Nanotropfens ist.

17. Einheit zur Detektion und Quantifizierung von Analyten für den Einsatz des Verfahrens nach einem beliebigen der vorstehenden Ansprüche 1 bis 13, die Folgendes umfasst:
- ein Substrat (10) mit einer strukturierten Oberfläche zur Aufnahme einer Stammlösung, die die Analyten von Interesse enthält;
- ein Steuerungsmittel (30) für die Verdampfung der Lösung in der Nachbarschaft (V_{T}) einer dreifachen Linie (T) Flüssigkeit/Substrat/Atmosphäre;
- eine Platte (40), die in Berührung mit der Lösungsprobe aufgelegt werden soll, um diese zwischen dem Substrat und der Platte einzuschließen;
- ein Mittel zur Analyse der Oberfläche mit Mikro- oder Nanostruktur des Substrats, **dadurch gekennzeichnet, dass** die Platte funktionalisiert und/oder strukturiert ist.

18. Einheit zur Detektion und Quantifizierung von Analyten nach Anspruch 17, **dadurch gekennzeichnet, dass** die Platte (40) stärker hydrophob ist, als das Substrat (10).

19. Einheit zur Detektion und Quantifizierung von Analyten nach einem beliebigen der vorstehenden Ansprüche 17 oder 18, **dadurch gekennzeichnet, dass** sie ferner ein oder mehrere Substrate (10a, 10b) umfasst, die jeweils eine funktionalisierte Oberfläche (20a, 20b) aufweisen, die spezifisch Analyten von Interesse (2a, 2b) fixiert.

20. Einheit zur Detektion und Quantifizierung von Analyten nach einem beliebigen der vorstehenden Ansprüche 17 bis 19, wobei das Analysemittel fähig ist, von der strukturierten Oberfläche des Substrats gefangene Analyten zu zählen.

21. Einheit zur Detektion und Quantifizierung von Analyten nach einem beliebigen der vorstehenden Ansprüche 17 bis 20, wobei das Analysemittel fähig ist, die vorher erzielte Anzahl Analyten mit einer Umwandlungbstabelle zu vergleichen, um die Analytenkonzentration in der Stammlösung zu ermitteln.

22. Einheit zur Detektion und Quantifizierung von Analyten nach einem beliebigen der vorstehenden Ansprüche 17 bis 21, wobei das Substrat (10) und die Platte (40) beweglich und deutlich parallel zueinander in einer Translationsrichtung (F2a-F2b) montiert sind.

23. Einheit zur Detektion und Quantifizierung von Analyten nach einem beliebigen der vorstehenden Ansprüche 17 bis 21, wobei das Substrat (10) und die Platte (40) beweglich zueinander in einer Translationsrichtung (F2a-F2b) montiert sind, wobei die Platte (40) dem Substrat (10) gegenüber geneigt montiert ist, so dass sie die Lösungsprobe auf das strukturierte Substrat aufbringt.

24. Einheit zur Detektion und Quantifizierung von Analyten nach einem beliebigen der vorstehenden Ansprüche 17 bis 23, wobei die Platte (40) flexibel ist.

25. Einheit zur Detektion und Quantifizierung von Analyten nach einem beliebigen der vorstehenden Ansprüche 17 bis 24, die ferner eine Umschließung (50) unter Schutzatmosphäre umfasst, die das Substrat und die Probe umgibt.

26. Einheit zur Detektion und Quantifizierung von Analyten nach Anspruch 25, wobei die Umschließung einen Partialdruckregler (60) der Komponenten der Flüssigkeit in der Atmosphäre umfasst.

27. Einheit zur Detektion und Quantifizierung von Analyten nach einem beliebigen der vorstehenden Ansprüche 25 oder 26, die ferner Pump- und/oder Injektionskanäle für Gas oder ein Gasgemisch umfasst.

28. Einheit zur Detektion und Quantifizierung von Analyten nach einem beliebigen der vorstehenden Ansprüche 17 bis 27, die ferner eine Vorrichtung für die Einleitung von thermischer und/oder elektromagnetischer Energie umfasst.

29. Einheit zur Detektion und Quantifizierung von Analyten nach einem beliebigen der vorstehenden Ansprüche 17 bis 28, wobei das Steuermittel (30) mit zumindest einer Vorrichtung zur Beobachtung der dreifachen Linie (T) gekoppelt ist, um die Steuerung der Verdampfung anzupassen und die Bewegungsgeschwindigkeit der dreifachen Linie auf der strukturierten Oberfläche des Substrats auf zumindest einen gewünschten Wert einzustellen.

30. Einheit zur Detektion und Quantifizierung von Analyten nach einem beliebigen der vorstehenden Ansprüche 26 bis 29, wobei der Partialdruckregler (60) mit zumindest einer Vorrichtung zur Beobachtung der dreifachen Linie (T) gekoppelt ist, um die Partialdrücke der Komponenten der Flüssigkeit in der Atmosphäre anzupassen und die Bewegungsgeschwindigkeit der dreifachen Linie (T) auf zumindest einen gewünschten Wert einzustellen.

31. Einheit zur Detektion und Quantifizierung von Analyten nach einem beliebigen der vorstehenden Ansprüche 17 bis 30, wobei die Oberfläche des Substrats eine Struktur umfasst, die aus topographischen, biologischen, chemischen, elektrostatischen, magnetischen Strukturierungen oder einer Kombination davon gezogen ist.

32. Einheit zur Detektion und Quantifizierung von Analyten nach einem beliebigen der vorstehenden Ansprüche 17 bis 31, wobei die Stammlösung eine kolloidale Lösung, eine vorkonditionierte Lösung, eine vorgefilterte Lösung, eine Tenside und Kalibrierungsziele enthaltende Lösung, eine Lösung mit farblich, durch Fluoreszenz oder durch einen integrierten Balkencode markierten Zielen, oder eine Lösung mit Koppelung von Zielen und Sonden umfasst, die schon in Lösungen realisiert worden sind.

## Claims

1. A method of detecting and quantifying target analytes of interest (2) in a specimen (1) of liquid obtained from a parent solution, said parent solution having an unknown concentration of analytes to be determined, the liquid being able to evaporate in an atmosphere (Atm) in specified conditions of evaporation, the method comprising the following steps:
b1) depositing the specimen (1) on a substrate (10) having a micro- or nano-structured surface (20) defining analyte capture probes, so that the liquid specimen at least partially covers the structured surface of the substrate;
b2) depositing a functionalized and/or structured plate (40) in contact with the liquid specimen (1), for enclosing the latter between the substrate (10) and the plate (40);
c) causing controlled evaporation (5) of the specimen in the vicinity (V_{T}) of a liquid/substrate/atmosphere triple line (T), constituted by the interface between the liquid of the specimen, the atmosphere and the substrate, in such a way that this triple line moves, at a controlled speed, over the structured surface of the substrate, as the liquid evaporates into the atmosphere, and so that the target analytes are captured, by convective, capillary assembly directed toward the probes;
d) analyzing the structured surface of the substrate obtained after step c).

2. The method of detecting and quantifying analytes as claimed in claim 1, in which step d) is applied by counting the analytes captured at step c) by the micro- or nano-structured surface of the substrate, and by comparing the number of target analytes captured against a conversion table, to obtain the concentration of analytes in the parent solution.

3. The method of detecting and quantifying analytes as claimed in claim 1, in which step d) is applied by counting the analytes captured at step c), said analytes comprising reference analytes whose concentration is known and analytes of interest whose concentration is unknown but proportional to that of the reference analytes, and by comparing the proportions of the reference analytes and of the analytes of interest.

4. The method of detecting and quantifying analytes as claimed in one of the preceding claims, in which the plate (40) and the substrate (10) are displaced relative to one another in a direction of translation (F2a-F2b) roughly parallel to the substrate, during controlled evaporation of the specimen.

5. The method of detecting and quantifying analytes as claimed in one of the preceding claims, in which the substrate and the specimen are confined in an enclosure (50) with controlled atmosphere.

6. The method of detecting and quantifying analytes as claimed in claim 5, in which the partial pressure of the components of the liquid in the controlled atmosphere is regulated during step c).

7. The method of detecting and quantifying analytes as claimed in any one of the preceding claims, in which step c) is applied by supplying an amount of energy sufficient to cause and control the evaporation of the liquid at the triple line.

8. The method of detecting and quantifying analytes as claimed in any one of the preceding claims, comprising a step a) of pre-conditioning of the parent solution.

9. The method of detecting and quantifying analytes as claimed in claim 8, in which the pre-conditioning of the parent solution consists of removing unwanted analytes from the parent solution, of adding new target analytes, and/or of adding solvents or new molecules promoting convective, capillary assembly on the structured surface.

10. The method of detecting and quantifying analytes as claimed in any one of the preceding claims, comprising a preliminary step of preparation of the structured surface consisting of depositing a droplet of liquid comprising probe molecules, specific to the target analytes, on the surface (20) of the substrate (10), so that the droplet at least partially covers the surface of the substrate, then causing controlled evaporation of the droplet in the vicinity (V_{T}) of a droplet/substrate/atmosphere triple line (T), in such a way that this triple line moves, at a controlled speed, over the structured surface of the substrate, as the liquid evaporates into the atmosphere, and so that the probe molecules attach to the surface of the substrate to create a probe network structuring the surface of the substrate.

11. The method of detecting and quantifying analytes as claimed in any one of the preceding claims, comprising an intermediate step between step c) and step d), of fixation of fluorophores on the captured target analytes to permit counting by fluorometry during step d).

12. The method of detection and quantification as claimed in any one of the preceding claims, further comprising, between step c) and step d), at least one step of specific differentiation of the analytes trapped (2a, 2b) at step c), by applying the substrate (10) obtained after step c) on one or more functionalized capture surfaces (20a, 20b).

13. The method of detection and quantification as claimed in any one of the preceding claims, in which, during step c), evaporation is controlled in such a way that the triple line (T) moves at a constant speed.

14. The method of detection and quantification as claimed in any one of claims 1 to 12, in which, during step c), evaporation is controlled in such a way that the triple line (T) moves at a variable speed.

15. The method of detection and quantification as claimed in any one of the preceding claims, in which the probes of the surface (20) each define a grating providing an optical spectrum, step d) being performed by analyzing the optical spectrum of each grating after capture of the analytes at step c).

16. The method of detection and quantification as claimed in any one of claims 1 to 14, in which the probes of the surface (20) are cavities of different sizes, step c) resulting in capture of the target analytes in micro- or nano-droplets of different sizes, trapped in cavities of different sizes, and step d) being applied by measuring variations in intensity of at least one physical or chemical property of each micro- or nano-droplet, by determining the volume of the "limit" micro- or nano-droplet which does not contain any analyte, the concentration of the analyte in the parent solution being equal to 1 divided by the volume of the limit micro- or nano-droplet.

17. An assembly for detecting and quantifying analytes for implementing the method as claimed in any one of claims 1 to 13, comprising
- a substrate (10) having a structured surface intended for receiving a specimen of parent solution containing the analytes of interest;
- a means of control (30) of the evaporation of the solution in the vicinity (V_{T}) of a liquid/substrate/atmosphere triple line (T);
- a means of analyzing the micro- or nano-structured surface of the substrate.

18. The assembly for detecting and quantifying analytes as claimed in claim 17, **characterized in that** the plate (40) is more hydrophobic than the substrat (10).

19. The assembly for detecting and quantifying analytes as claimed in claim 17 or 18, **characterized in that** it comprises one or more substrats (10a, 10b) having each a functionalized surface (20a, 20b)likely to specifically fix analytes (2a, 2b) of interest.

20. The assembly for detecting and quantifying analytes as claimed in claims 17 to 19, in which the means of analysis is able to count analytes trapped by the structured surface of the substrat.

21. The assembly for detecting and quantifying analytes as claimed in claims 17 to 20, in which the means of analysis is able to compare the number of analytes obtained previously against a conversion table, to obtain the concentration of analytes in the parent solution.

22. The assembly for detecting and quantifying analytes as claimed in claims 17 to 21, in which the substrate (10) and the plate (40) are mounted movably and roughly parallel to one another in a direction of translation (F2a-F2b).

23. The assembly for detecting and quantifying analytes as claimed in claims 17 to 21, in which the substrate (10) and the plate (40) are mounted movably relative to one another in a direction of translation (F2a-F2b), the plate being inclined relative to the substrate so as to apply the specimen of solution on the structured substrate.

24. The assembly for detecting and quantifying analytes as claimed in claims 17 to 23, in which the plate (40) is flexible.

25. The assembly for detecting and quantifying analytes as claimed in one of claims 17 to 24, further comprising an enclosure (50) with controlled atmosphere surrounding the substrate and the specimen.

26. The assembly for detecting and quantifying analytes as claimed in claim 25, in which the enclosure comprises a regulator (60) of partial pressure of the components of the liquid in the atmosphere.

27. The assembly for detecting and quantifying analytes as claimed in one of claims 25 or 26, further comprising channels for pumping and/or injecting a flow of gas or of gas mixture.

28. The assembly for detecting and quantifying analytes as claimed in any one of claims 17 to 27, further comprising a device for supplying thermal and/or electromagnetic energy.

29. The assembly for detecting and quantifying analytes as claimed in any one of claims 17 to 28, in which the control means (30) is coupled to at least one device for observation of the triple line (T) for adapting the control of evaporation and adjusting the speed of displacement of the triple line to at least one desired value, on the structured surface of the substrate.

30. The assembly for detecting and quantifying analytes as claimed in claims 26 to 29, in which the regulator of partial pressure (60) is coupled to at least one device for observation of the triple line (T) for adapting the partial pressures of the components of the liquid in the atmosphere and for adjusting the speed of displacement of the triple line (T) to at least one desired value.

31. The assembly for detecting and quantifying analytes as claimed in any one of claims 17 to 30, in which the surface of the substrate comprises a structuring selected from topographic, biological, chemical, electrostatic, magnetic structurings or a combination of these structurings.

32. The assembly for detecting and quantifying analytes as claimed in any one of claims 17 to 31, in which the parent solution is a colloidal solution, a preconditioned solution, a pre-filtered solution, a solution that has surfactants and calibration targets, a solution incorporating targets labeled by color, by fluorescence or by an integrated barcode, or a solution incorporating target-probe couplings already effected in solutions.
